# EUROPEAN PATENT SPECIFICATION

(11) **EP 1 345 587 B1**
(45) Date of publication and mention of the grant of the patent: **26.08.2009**
(21) Application number: 01271212.1
(22) Date of filing: 18.12.2001
(51) Int. Cl.: A61K 9/00, A61K 47/34, A61K 47/36, A61K 47/10, A61K 9/14

(54) **IONOPHORE ANTIBIOTIC FORMULATIONS**
IONOPHORE ANTIBIOTISCHE FORMULIERUNGEN
PREPARATIONS D'UN ANTIBIOTIQUE IONOPHORE

(30) Priority: 19.12.2000 NZ 50909200; 13.09.2001 NZ 51418301
(43) Date of publication of application: 24.09.2003
(73) Proprietor: Eli Lilly & Company( NZ) Limited, Manukau City, Auckland South (NZ)
(72) Inventor: AGNEW, Kim, Ewing, Melville, Auckland (NZ); HEWITT, William, Austin, Auckland (NZ); HANHAM, Craig, Richard, Auckland (NZ); PURDY, Kevin, Grant, Auckland (NZ)
(74) Representative: Kent, Lindsey Ruth
(86) International application number: PCT/NZ2001/000290
(87) International publication number: WO 2002/049609

(56) References cited:
- WO-A-97/03650
- WO-A-98/16663

## Description

The present invention relates to ionophore antibiotic compositions and particularly, but not solely, to ionophore antibiotic compositions capable of dilution with water, suitable for inclusion directly or via a holding tank in the drinking water of an animal to have administered and/or self administered an ionophore antibiotic or ionophore antibiotics.

### BACKGROUND

Administration of ionophore antibiotics such as monensin to animals (preferably ruminants) is known to achieve in appropriate dosages advantages for a number of different purposes. These include the treatment or prevention of ketosis and/or bloat, the enhancement of milk production, enhancement of milk protein content in milk, enhancement of mineral uptake, enhancement of weight gain, and/or enhancement of feed conversion efficiency (in ruminants), desirable reproduction advantages and as a milk replacement. See US Patent 3,829,557.

In this respect we refer to European Patent Specification 0 139 595 A2 of KOFFOLK (1949) LTD which relates to a liquid ionophore antibiotic composition for ruminants and poultry where the antibiotic is dissolved in a non-toxic water-soluble organic solvent rather than a water soluble organic solvent, and in use the resulting solution is admixed with a liquid feed, a liquid vitamin concentrate or drinking water. There are claims for stability on standing.

The composition of EP 0 139 595 indicates that because monensin and its sodium salt is only slightly soluble in water that it is generally administered in a dry form in an animal feed and/or in dry liquid milk replacer compositions. The same is also indicated as being true for another ionophore antibiotic lasalocid which in US Patent 3,715,372 is reported to be completely insoluble in water.

The composition of EP 0 139 595 uses as an organic solvent for the ionophore antibiotic a solvent selected from the group comprising propylene glycol, glycerol, ethanol and isopropanol and mixtures thereof.

Example 1 of EP 0 139 595 indicates that 250 g of a mycelium containing 10% monensin was mixed at room temperature with 1250 cc propylene glycol for 2 hours by exposing the mixture to ultrasound. There is an indication that 50% of the monensin was found in solution in the propylene glycol.

Monensin is usually available commercially as the sodium salt of the acid. Monensin sodium is available in two forms, namely, a crystalline form or a mycelial form. The mycelial form has only about a 20% activity while the crystalline form has greater than 90% active monensin sodium.

Reference herein to "monensin" where the context allows encompasses all forms thereof including monensin, alkali metal salts of monensin and monensin-esters and includes mixtures. Likewise for the other ionophore antibiotics.

In our New Zealand Patent Specification 272574/272940 (equivalent to PCT/NZ96/00068, WO 97/03650) we disclose an aqueous base suspension concentrate of an ionophore antibiotic or ionophore antibiotics such as monensin.

In one aspect in NZ 272574/272940 that invention is defined as an aqueous base suspension concentrate of an ionophore antibiotic or ionophore antibiotics capable of aqueous dilution (if desired) and capable (with or without such aqueous dilution) of being orally administered to an animal by an active dosing regime (eg; by drenching), said concentrate comprising or including
(I) at least one ionophore antibiotic in
(II) an aqueous system containing
   (i) a wetting and/or surfactant agent, (preferably alkyl polyglycoside)
   (ii) an antifreeze agent or agents in which the ionophore antibiotic or antibiotics is or are no more than sparingly soluble,
   (iii) a suspension agent (eg gum (s)),
   (iv) optionally, an antifoam agent or system,
   (v) optionally, a preservative,
   (vi) optionally, a de-bittering agent,
   (vii) optionally, a pH buffering system, and
   (viii) water.

The preferred ionophore antibiotic of NZ 272574/272940 is sodium monensin. Preferably an antifreeze agent or agents in which the ionophore antibiotic(s) is no more than sparingly soluble (such as a glycol or polyglycol) is present. Additional, preferably an antifoam agent is present (e.g. simethicone and a particulate carrier such as silicon dioxide therefor).

NZ 272574/272940 also discloses a method of drenching an animal with such an ionophore antibiotic which involves administering orally to such an animal a diluted form of the compositions, such dilution being with water.

We have found monopropylene glycol to be a poor solvent of monensin. While we have in NZ 272574/272940 referred to monopropylene glycol as being an antifreeze agent in which the ionophore antibiotic is sparingly soluble, we do appreciate that solubility to the extent referred to in Example 1 of EP 0 139 595 may be achieved when induced with ultrasound or heating.

The form of crystalline monensin used for the examples in NZ 272574/272940 has a mean particle size of the order of about 40 µm (microns) and in order to hold such large particles in suspension an aggressive supporting system for that purpose has been required.

Thus despite the advances represented by the invention disclosed in NZ 272574/272940 (providing compositions useful for an active system of administering ionophore antibiotics to ruminants), and despite assertions of EP 0 139 595, there continues to be a substantial need for compositions containing ionophore antibiotics which are inherently poorly soluble or insoluble in aqueous based systems, suitable for passive system administrations to ruminants. Such compositions must, to avoid under-dosing or toxic dosing, remain substantially homogeneous for extended periods of time.

The present invention investigates alternatives to such aggressive systems and targets a dosable composition capable of dispersion stability as a suspension in highly dilute drinking water for animals.

The present invention relies upon both a preferred smaller mean particle size of the ionophore antibiotic and an associated regime including related methods of manufacture, uses etc.

The present invention also recognises *inter alia* the therapeutic, energy and productivity benefits were monensin or any other suitable ionophore antibiotic (see the listing in our aforementioned patent specification, and below) to be used as a trough treatment.

### Trough Treatment Systems

Currently three types of trough treatment system for animal drinking water and not hitherto used with an ionophore antibiotic are known.

Three types are in use:
1. Proportional feed systems e.g. that branded DOSATRON^{™} delivers a % mix in proportion to demand (from 0.2-2% for dosatron). Aim is to spread delivery of the complete batch over 24 hours.
2. Dump systems Continually delivers drench into lines regardless of demand. Tends to be all used up within 5 hours. -
3. Individual trough systems e.g. PETA^{™} dispenser Delivers concentrated drench in relation to demand but pay out is skewed early on.

The present invention includes an ionophore antibiotic base composition capable of aqueous dilution which may be useful in such a trough treatment system. The object of the present invention is to provide such a composition and/or to provide drinking water for an animal to allow administration of an ionophore antibiotic to the animal.

We have determined that a microfine form of an ionophore antibiotic with its smaller mass to surface area ratio is a significant advantage over less fine (eg; 40 µm (microns) mean particle size) ionophore antibiotic inclusions in an aqueous composition and more so when it is to be used in such a way where it is subjected to unsupervised dilution, e.g.; to provide an infeed (directly or indirectly) into a dilution quantity of trough make up water. Such advantages to uniform suspendability we believe to be equally applicable to the more preferred crystalline forms as well as the less preferred mycellial forms of the ionophore(s).

We have also determined that a suitable glycol such as monopropylene glycol can be used in such a way to pre-prepare an ionophore antibiotic for subsequent suspension as a aqueous concentrate and thereafter to carry at least an antifreeze advantage over to its subsequent use (e.g.; as an infeed aqueous concentrate for a dose-certain water system) without leading to crashing out of the ionophore antibiotic. Glycols (such as monopropylene glycol) unless agressively treated provide no significant uptake of the stably suspended ionophore antibiotic (such as monensin) from an aqueous system.
This we consider desirable since an organic uptake can lead to crashing out on dilution. For example, an organic solvent, such as methanol, allows monensin to crash out of solution if any water is added.

In the preferred forms of the present invention the ionophore antibiotic of choice is monensin in any of its appropriate forms (eg; sodium monensin). Other ionophore antibiotics however fall within the scope of the present invention and these include Lonomycin, Ionomycin, Laidlomycin, Nigericin, Grisorixin, Dianemycin, Lenoremycin, Salinomycin, Narasin, Antibiotic X206, Alborixin, Septamycin, Antibiotic A204, Maduramicin and Semduramicin, Compound 47224, Lasalocid (also including factors A, B, C, D and E), Mutalomycin, Isolasalocid A, Lysocellin, Tetronasin, Echeromycin, Antibiotic X-14766a, Antibiotic A23187, Antibiotic A32887, Compound 51532 and K41.

### STATEMENTS OF THE INVENTION

In one aspect, the present invention provides an aqueous suspension of at least one ionophore antibiotic capable of subsequent dilution, said suspension comprising:
a milled product comprising an ionophore antibiotic or ionophore antibiotics milled with at least the following dispersing agents:
   (i) a glycol and
   (ii) at least one of a lignosulfonate and a polyglycoside,
      wherein the ionophore antibiotic or ionophore antibiotics have a mean particle size of less than 20 µm (microns), and
      at least water and a suspension agent selected from xanthan gum and guar gum.

In another aspect the present invention consists in a method of forming an aqueous suspension of at least one ionophore antibiotic capable of subsequent dilution, said method comprising:
milling an ionophore antibiotic or ionophore antibiotics (preferably in a crystalline form) with at least the following dispersing agents:
   (i) a glycol and
   (ii) at least one of a lignosulfonate and a polyglycoside,
wherein the ionophore antibiotic or ionophore antibiotics are reduced to a mean particle size of less than 20 µm (microns) and the milling is in liquid supported conditions and
subsequently formulating the suspension with at least water and a suspension agent selected from zanthan gum and guar gum.

Preferably said ionophore antibiotic is of sufficient purity as to minimise any requirement for an anti foaming agent but if an anti foaming agent is necessary, preferably said anti foaming agent is a GENSIL^{™} system, i. e. of simethicone/silicon dioxide support for the simethicone.

Preferably said suspension includes a buffer system.

Preferably said suspension includes a preservative.

Where reference is made to a glycol preferably said glycol is a liquid and preferably said glycol assumes an anti freezing role in the resultant aqueous suspension.

Example of a suitable glycol is monopropylene glycol but other examples include ethylene glycol, diethylene glycol and dipropylene glycol.

Preferably the ionophore antibiotic or ionophore antibiotics is at least primarily of a crystalline form but in other forms it can in part be of the mycelial form. For example, if as is preferred, the ionophore antibiotic is monensin (e. g. present for example as sodium monensin), preferably the ionophore antibiotic is substantially free of the mycelial form. Were the mycelial form to be utilised however, preferably there are commensurate changes in the inclusion to reflect the lesser activity of that form or an additional input to any dilution system to achieve appropriate activities.

Preferably said milling is in liquid supported conditions (preferably as a result of a liquid glycol inclusion), preferably with a minimum of water necessary for the purpose (if any), and preferably is such as to reduce the antibiotic to a microfine form.

In the present invention, the ionophore antibiotic is reduced to a mean particle size less than 20 µm (microns).

Most preferably the microfine condition of the ionophore antibiotic is to a mean particle size less than 5 µm (microns).

Most preferably the mean particle size resulting from the milling procedure or procedures (can be a single or multiple milling procedures) is such as to provide a mean particle size of the preferably crystalline ionophore antibiotic (e. g. monensin) in a range of from 0. 1 to 1.0 µm (microns).

The milling stage or stages involves milling with a suitable polyglycoside or lignosulfonate, or both. One such polyglycoside is alkyl polyglycoside. One possible lignosulfonate compound is ULTRAZINE NA^{™} or BORRESPERSE NA^{™} [of Borregaard Industries Ltd of Norway] (most preferably ULTRAZINE NA^{™}).

Optionally GENSIL^{™} or another suitable antifoam agent is also milled with the crystalline monensin or other antibiotic.

Preferably the milling of all coating agents is simultaneous although it can be in part or totally serially.

In a preferred mix the ionophore antibiotic is milled simultaneously with liquid monopropylene glycol and a suitable polyglycoside and/or lignosulfonate compound (more preferably a lignosulfonate) having a"wetting"function and an antifoam agent (optionally) and some water.

In a further aspect the present invention consists in an aqueous suspension formed by one or more of the methods of the present invention.

Herein disclosed is a method of forming an aqueous suspension capable of subsequent dilution, said method including prior to any substantial presence of water and/or a suspension agent (such as a suitable gum), milling the ionophore antibiotic or ionophore antibiotics (preferably in a crystalline form) with at least a suitable glycol.

Preferably said milling is to a mean particle size very much less than 40 µm (microns).

Preferably the mean particle size is less than 20 µm (microns), more preferably less than 5 µm (microns) and most preferably into a mean particle size of from 0.1 to 1.0 µm (microns).

Said milling includes the presence of a suitable lignosulfonate and/or a suitable polyglycoside.

Preferably said milling includes the presence of an antifoam agent.

Preferably said milling includes at least some water.

Preferably no suspension agent (eg; a gum typified by xanthan gum or guar gum) is present at said milling.

Preferably said method is performed substantially as hereinafter described with reference to the accompanying drawing.

Preferably the product includes as its suitable glycol monopropylene glycol.

Preferably the aqueous suspension is of a kind hereinafter described which includes monopropylene glycol, an additional wetting agent (eg; a lignosulfonate or polyglycoside or both) and a suspension agent.

Preferably the ionophore antibiotic is monensin and preferably that monensin is in a crystalline form.

Herein disclosed is a method of forming an aqueous suspension of at least one ionophore antibiotic capable of subsequent dilution, said method including milling the ionophore antibiotic or ionophore antibiotics (preferably in a crystalline form) with at least a suitable polyglycoside or a suitable lignosulfonate.

Preferably said method performs part of a method as previously defined.

Herein disclosed is an aqueous ionophore antibiotic suspension capable of further dilution without any substantial crashing out of the ionophore inclusion, said aqueous concentrate comprising or including
an ionophore antibiotic,
monopropylene glycol (or other suitable glycol),
a further material having a wetting characteristic,
a suspension agent,
optionally an antifoam agent, and water,
   wherein at least the monopropylene glycol and the ionophore antibiotic have been milled together prior to mixing with the water or at least any substantial amount of the water.

The further material having a wetting characteristic is a suitable polyglycoside or lignosulfonate.

Preferably said suitable polyglycoside is alkyl polyglycoside although more preferably the additional wetting agent is a lignosulfonate typified by ULTRAZINE NA^{™} or BORRESPERSE NA^{™} (more preferably ULTRAZINE NA^{™}).

Preferably the ionophore antibiotic is a crystalline form rather than a mycellial form.

Preferably the ionophore antibiotic is monensin or a monensin.

Preferably the ionophore antibiotic at least post milling has a particle size less than 5 µm (microns) and more preferably below 2 µm (microns).

Preferably the particle size range of the antibiotic is from 0.1 µm (microns) to 1.0 µm (microns).

Preferably the aqueous ionophore antibiotic suspension remains substantially homogeneous for a period longer than 7 days; more preferably for a period longer than 24 days.

It does not matter whether or not the milling reduces the ionophore antibiotic to the requisite mean particle size or whether or not it is already milled almost to that size. What is important is to have resultant particles of that size appropriately coated with the "wetting" agents which includes preferably a multifunctional glycol (eg; monopropylene glycol) and preferably an additional wetting agent.

Herein disclosed is an aqueous ionophore antibiotic suspension comprising or including
up to 20% w/v of a microfine (i. e. less than 20 µm (micron) mean particle size) crystalline ionophore antibiotic,
2 to 20% w/v of monopropylene glycol,
up to 10% w/v of a wetting agent,
0.1 to 5% w/v suspension agent, and water.

Preferably the aqueous ionophore antibiotic suspension remains substantially homogeneous for a period longer than 7 days; more preferably for a period longer than 24 days.

Preferably the formulation is substantially as described in any one of Examples 1 to 5.

Herein disclosed is a composition suitable for providing a direct in feed and/or indirect in feed (e. g. via a make up tank) into a water system from which target animal can drink, said composition being an aqueous composition of (at least)
(a) microfine ionophore antibiotic or microfine ionophore antibiotics,
(b) a glycol in which the ionophore antibiotic(s) is or are no more than sparingly soluble,
(c) a wetting agent,
(d) a suspension agent, and
(e) water, and
   optionally any one or more of
   an antifoam agent or system,
   a preservative,
   a debittering agent, and
   a pH buffering system.

Preferably the aqueous ionophore antibiotic suspension remains substantially homogeneous for a period longer than 7 days; more preferably for a period longer than 24 days.

Preferably said suspension agent is xanthan gum.

Preferably said wetting and/or surfactant agent (c) is a lignosulphonate (e: g. ULTRAZINE NA^{™}) or a polyglycoside (preferably alkyl polyglycoside).

Preferably said ionophore antibiotic is in either a crystalline or mycellial form or both.

Preferably a crystalline form is utilised.

Preferably said ionophore antibiotic is monensin (e. g. sodium monensin).

Preferably said microfine ionophore antibiotic is of a particle size less than 5 µm (microns) (preferably less than 2 µm (microns)) and preferably has a mean particle size in the range of from 0.1 to 1.0 µm (microns).

Preferably xanthan gum is present and in a quantity greater than 0.16 w/v% and preferably about 0.4 w/v% (ie, the amount required being more for the more concentrated aqueous concentrate, eg; will unlimited dilute none may be required).

Preferably the mode of mixing and formulation is substantially as disclosed herein with a preferred composition being substantially as follows (wherein preferably the mean sodium monensin particle size is substantially 5 µm (microns)).

| **Ingredient (common/chemical name)** | **Quantity (% w/w)** | **Function** |
|---|---|---|
| Sodium Monensin | 6.33% (about 6% monensin) w/v | Ionophore antibiotic |
| Monopropylene glycol | 10% w/v | Antifreeze |
| Alkyl Polyglycoside or | 0.5% w/v | Surfactant/Wetting agent |
| ULTRAZINE NA^{™} | 4-5% w/v | |
| Disodium Phosphate Anhydrous | 0.355% w/v | Buffer |
| MonoPotassium phosphate Dihydrate | 0.04% w/v | Buffer |
| Dialkyl dimethyl ammonium bromide | 0064% w/v | Preservative |
| Xanthan Gum | 0.4% w/v | Suspension agent |
| Simethicone | 0.333% w/v | e. g. GENSIL^{™} |
| Silicon Dioxide | 0.167% w/v | Antifoam system |
| Water | to 100% | |

An alternative preferred formulation is (wherein preferably the mean sodium monensin particle size is substantially 5 µm (microns)).

| **Ingredient name (common or chemical)** | **Quantity (%w/w)** | **Function** |
|---|---|---|
| Sodium Monensin QA 166H | 6.747% | Active |
| Monopropylene glycol | 10% | Antifreeze |
| Didecyl dimethyl Ammonium Bromide | 0.1% | Preservative |
| Xanthan Gum | 0. 5% | Suspension agent |
| Sodium lignosulphonate | 4.1% | Wetting agent |
| Alkyl Polyglycoside | 3.8% | Wetting agent |
| Simethicone | 0.333% | Antifoam |
| Silicon Dioxide | 0.167% | Antifoam |
| Water balance q.v | 74.47% | Diluent |

Herein disclosed is a composition for inclusion in a water trough fed in system being a composition as previously defined which without dilution is adapted to be in feed into the water supply.

Herein disclosed is a drinking water supply for an animal (preferably a ruminant animal) which has an in feed therein of a composition as previously defined.

Herein disclosed is a method of providing an ionophore antibiotic to a target mammal which comprises providing to the animal drinking water with a dispersed ionophore antibiotic, said ionophore antibiotic having been included in the water supply by an intake from a composition as previously defined.

Herein disclosed is an ionophore antibiotic composition capable of dilution with water to a substantially stable dispersed form in all water then present, said composition comprising or including:
at least one ionophore antibiotic of a mean particle size of less than 20 µm (microns), and
at least one dispersing agent.

Preferably the mean particle size of at least one ionophore antibiotic is substantially 5 µm (microns).

Preferably the dispensed form in water remains substantially homogeneous for at least 24 days.

Preferably a liquid vehicle is or is also present which preferably is or includes water.

Preferably or alternatively said liquid vehicle is or includes one compatible liquid organic compound, preferably selected from mineral and vegetable oils.

Preferably the milling is in the absence of any suspension agent, or alternatively a suspension agent selected from the gums as previously described is present; preferably the suspension agent is one or more of xanthan gum, guar gum, acacia gum and a cellulose gum.

Preferably a liquid vehicle(s) was present at the time of milling of the ionophore antibiotic sufficient to reduce the consistency of the mill mix to a millable consistency.

Herein disclosed is animal drinking water having at least one particulate ionophore antibiotic substantially uniformly suspended therein, wherein the ionophore antibiotic is stably suspended.

Preferably the ionophore antibiotic remains stably suspended for at least 24 days.

Preferably particles of the ionophore antibiotic(s) are of a mean particle size of less than 10 µm (microns); more preferably they are of a mean particle size of substantially 5 µm (microns).

Preferably the drinking water is made by a proportioned mix dispensing there into of a more concentrated aqueous suspension of the ionophore antibiotic and dispersing agents present in that more concentrated aqueous suspension provides the substantially uniform dispersion of the ionophore antibiotic(s) in the drinking water.

Preferably the more concentrated aqueous suspension contains one or more suspension agents.

Herein disclosed is animal drinking water having at least one particulate ionophore antibiotic substantially uniformly suspended therein, wherein the particles of the ionophore antibiotics are of a mean particle size less than 10 µm (microns); more preferably the particle size of the ionophore antibiotics are of a mean particle size of substantially 5 µm (microns).

Preferably the ionophore antibiotic remains stably suspended for at least 24 days.

Herein disclosed is trough water accessible to an animal to drink, the water having a particulate ionophore antibiotic substantially uniformly suspended therein, wherein the ionophore antibiotic is stably suspended.

Preferably the ionophore antibiotic remains stably suspended for at least 24 days.

Preferably the particles of the ionophore antibiotic(s) is of a mean particle size of less than 10 µm (microns); more preferably the particles of the ionophore antibiotic(s) is of a mean particle size of substantially 5 µm (microns).

Preferably the trough water is made by a proportioned mix dispensing there into of a more concentrated aqueous suspension of the ionophore antibiotic and dispersing agents present in that more concentrated form provides the substantially uniform dispersion of the ionophore antibiotic(s) in the drinking water.

Preferably the more concentrated aqueous suspension contains one or more suspension agents.

Herein disclosed is trough water accessible to an animal to drink the water having a particulate ionophore antibiotic substantially uniformly suspended therein, wherein the ionophore antibiotic remains stably suspended for at least 24 days.

Preferably the particle size of the ionophore antibiotics are substantially 5 µm (microns).

Preferably the ionophore antibiotic remains stably suspended for at least 24 days.

Herein disclosed is a method of dispensing a particulate ionophore antibiotic into a body of drinking water which comprises or includes the steps of:
taking a composition comprising or including at least one ionophore antibiotic of mean particle size of less than 20 µm (microns) and at least one dispersing agent:
forming an aqueous suspension of the composition that has the ionophore antibiotic(s) substantially uniformly dispersed therein and which aqueous suspension is available for dispensing into animal drinking water, and
dispensing at a rate (continuously or continually), that aqueous suspension into the drinking water or a makeup supply of water thereto so as to provide the body of drinking water with a uniform dispersion of the ionophore antibiotic(s) therein which is within an acceptable imbibing concentration range for the animal or animals having access thereto.

Preferably the mean particle size of the at least one ionophore antibiotic is substantially 5 µm (microns).

Preferably said composition is in the form of a stable aqueous suspension prior to the forming of an aqueous suspension and the subsequent dispensing thereof into the drinking water or a make up supply of water.

A suspension agent selected from gums (as previously described) is present.

Preferably the ionophore antibiotic(s) remains stably suspended for at least 24 days.

Herein disclosed is a body of drinking water having a particulate ionophore suspended therein prepared by the method as previously described.

Herein disclosed is a method of forming a suspendable composition of at least one ionophore antibiotic and which suspendable composition is capable of subsequent dilution, said method including, prior to any optional presence of water and optional presence of a suspension agent, milling the ionophore antibiotic(s) with a suitable glycol.

Preferably said milling takes place in the presence of at least some liquid, which preferably includes water.

Preferably no gum is present.

Preferably said milling is to a mean particle size very much less than 20 µm (microns); more preferably the mean particle size is of about 5 µm (microns).

Preferably the milling takes place in the presence of a suitable lignosulfonate.

Preferably said milling takes place in the presence of a suitable Polyglycoside.

Preferably said milling takes place in the presence of a suitable antifoam agent.

There is provided a suspendable composition of at least one ionophore antibiotic prepared according to the method as previously described.

Herein disclosed is a method of forming a suspendable composition of at least one ionophore antibiotic and which suspendable composition is capable of subsequent dilution, said method including, prior to any optional presence of water and optional presence of a suspension agent, milling the ionophore antibiotic(s) with a suitable dispersion agent.

Preferably the milling takes place in the presence of a suitable lignosulfonate.

Preferably or alternatively said milling takes place in the presence of a suitable polyglycoside.

Said suitable dispersion agent is selected from the group consisting of a suitable lignosulfonate and a suitable polyglycoside.

Preferably the mean particle size is less than 20 µm (microns); more preferably less than 10 µm (microns); even more preferably the mean particle size is substantially 5 µm (microns).

Preferably said milling takes place in the presence of a suitable antifoam agent.

Preferably said milling takes place in the presence of at least some liquid.

A suitable glycol is present:
Preferably no gum is present.
Herein disclosed is a dose dispensable ionophore antibiotic composition comprising or including:
   at least one ionophore antibiotic,
   at least one dispersing agent,
   at least one suspension agent, and water,
      wherein the ionophore antibiotic(s) and at least one dispersing agent have been milled together in the presence of a liquid (which may be water or may include water but not necessarily so) and where the dispersing agent has been added post-milling in the presence of water.

The ionophore antibiotic is of a mean particle size of less than 20 µm (microns).

Preferably at least one suspension agent is or includes a gum and no such gum was present at the milling procedure.

Preferably some water was present at the milling procedure.

Preferably a polyglycoside was present as a dispersing agent at the milling stage.

Preferably a lignosulfonate was present as a dispersing agent at the milling stage.

A glycol was present as a dispersing agent at the milling stage.

Preferably an alkyl polyglycoside, a lignosulfonate and a propylene glycol are present at the milling stage.

Preferably any one or more of an antifoam agent or system, a preservative, a debittering agent and a pH buffering system is present.

Herein disclosed is a milled product useful in providing an aqueous suspension of at least one ionophore antibiotic, said product being the milled outcome of a mill mix of at least
at least one ionophore antibiotic, and
at least one dispersing agent,
   wherein the mill mix has been substantially free of suspension agents selected from the gums previously described and the mill mix has included at least one liquid component (which optionally can be the or one of the dispersing agent(s) or an additional material),
   and wherein, in the product, the process of milling has resulted in some physical association of the or at least one dispersing agent on the ionophore antibiotic particles, and wherein, in the product, the mean particle size of the ionophore antibiotic(s) is less than 20 µm (microns).

Preferably the mean particle size is substantially 5 µm (microns).

Herein disclosed is a suspendable composition for substantial dilution by water, said method comprising or including:
(I) milling at least one ionophore antibiotic and at least one dispersing agent so as to provide some physical association of the or at least one dispersing agent on ionophore antibiotic particles of a mean particle size of less than 20 µm (microns), and
(II) blending the post mill ionophore antibiotic(s)/dispersing agent(s) milled outcome with at least a suspension agent.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples.

### DEFINITIONS

Wherein the specification the following terms are used there are a number of possible alternatives, examples of which follow:

### Surfactants/Dispersants:

sorbitan esters, ethoxylated sorbitan esters, castor oil ethoxylates, ethoxylated fatty acids, ethoxylated alcohols, polyethylene glycol fatty acids, condensed napthalene sulphonic acid, glycerol esters, phosphate esters, sodium lauryl sulphate, sodium polyacrylate, ammonium polyacrylate, octyl phenyl ethoxylates, nonyl phenyl ethoxylates, quaternary ammonium compounds, sulphosuccinates, soya lecithin.

### Suspending aids, including gums:

aluminium stearate, silicon dioxide, modified clay including Smectite, Monmorillonite, Hectorite, Bentonite, dehydrogenated castor oil, titanium chelates, alkali soluble acrylic polymers, nonionic diurethanes, polyvinyl pyrollidone, polyvinyl alcohol, guar gum, gum arabic, hydroxy propyl methyl cellulose, hydroxy ethyl cellulose, sodium carboxy methyl cellulose, hydrophobically modified hydroxy ethyl cellulose.

### Defoamers/Antifoams:

polymethyl siloxane, unsaturated hydrocarbon oil, mineral oil, silica in oil type.

### Preservatives:

oxazolidines, benzothiazoles, benzalkonium chloride, substituted triazines, isothiazolones, hemiacetals, substituted benzoates, zinc pyridinethiol oxide, sodium pyridinethiol oxide.

### Buffering agents:

potassium tetroxalate, potassium hydrogen tartrate, potassium hydrogen phthalate, borax.

### DETAILED DESCRIPTION OF THE INVENTION

Preferred forms of the present invention will now be described with reference to the accompanying drawings in which
Figure 1: shows one mixing procedure utilised in accordance with the invention,
Figure 2: shows a second mixing procedure utilised in accordance with the invention, and
Figures 3A-3C: illustrate a DOSTATRON^{™} trough treatment system in three different modes of operation.

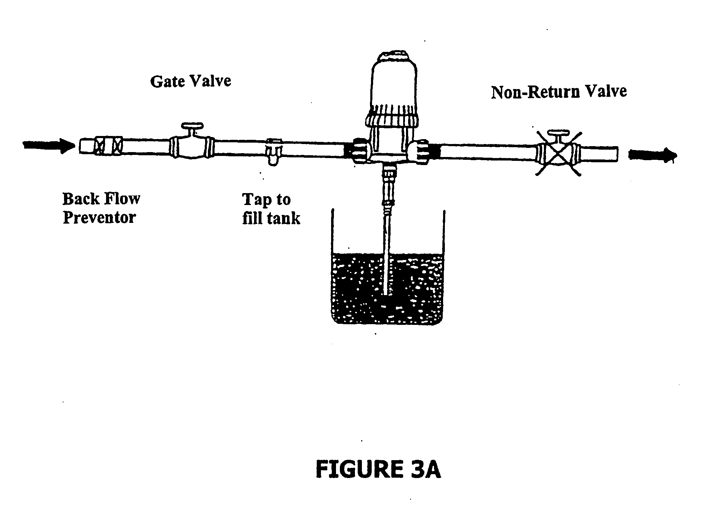

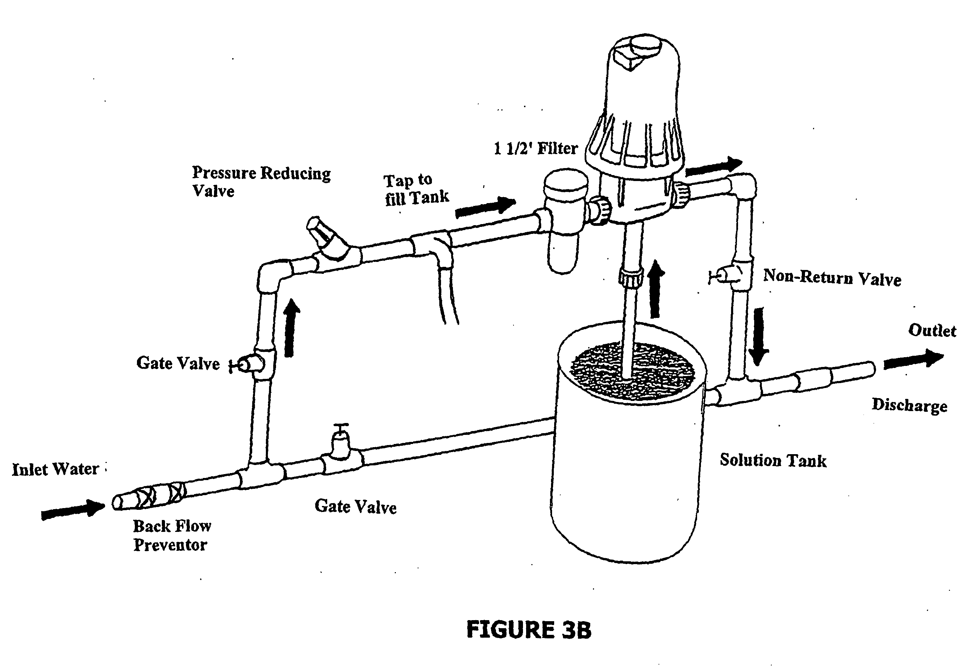

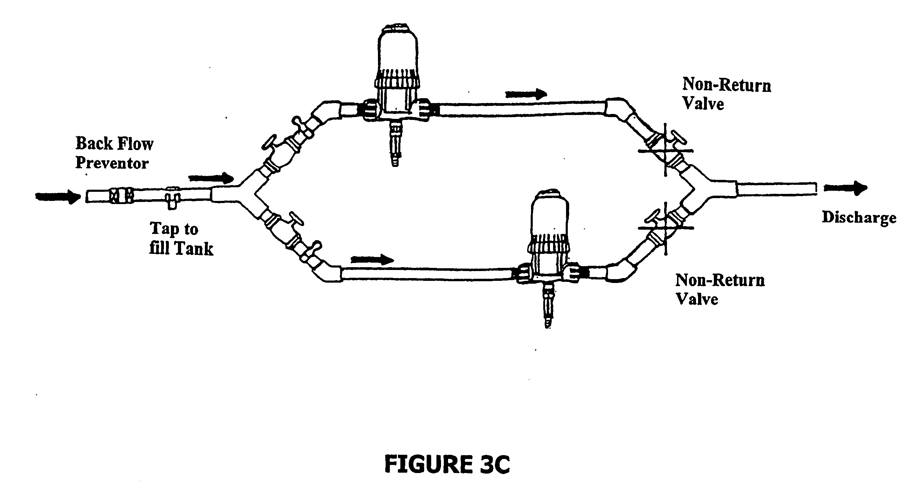

Shown in figures 3A to 3C are three installations of a DOSATRON^{™} type installation used for dosing materials into drinking water. The DOSATRON^{™} apparatus is recommended as being usable in line (figure 3A), on a bypass line (figure 3B) and in parallel (figure 3C). In this respect see the publicity materials available from the New Zealand distributor Mark Bell-Booth Ltd.

The DOSATRON^{™} apparatus itself employees a dosing piston driven by a volumetric hydraulic piston motor. The spread rhythm of the motor is proportional to the flow of water passing through the system and thus the rate of injection will likewise remain proportional in its reciprocating motion.

The apparatus has the capability of a wide range of daily dose settings and the parallel arrangement shown in figure 3 allows these to be doubled owing to the use of two DOSATRON^{™} dispensing units.

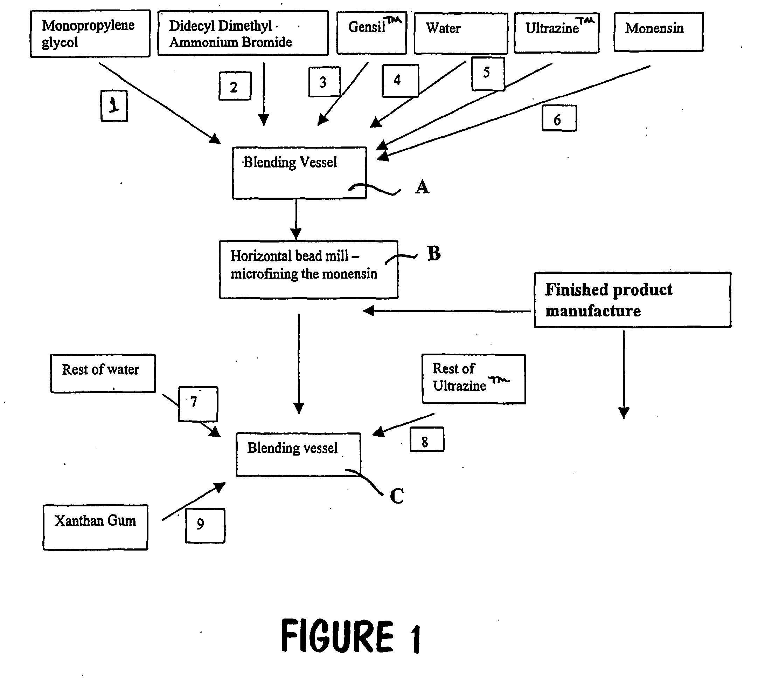

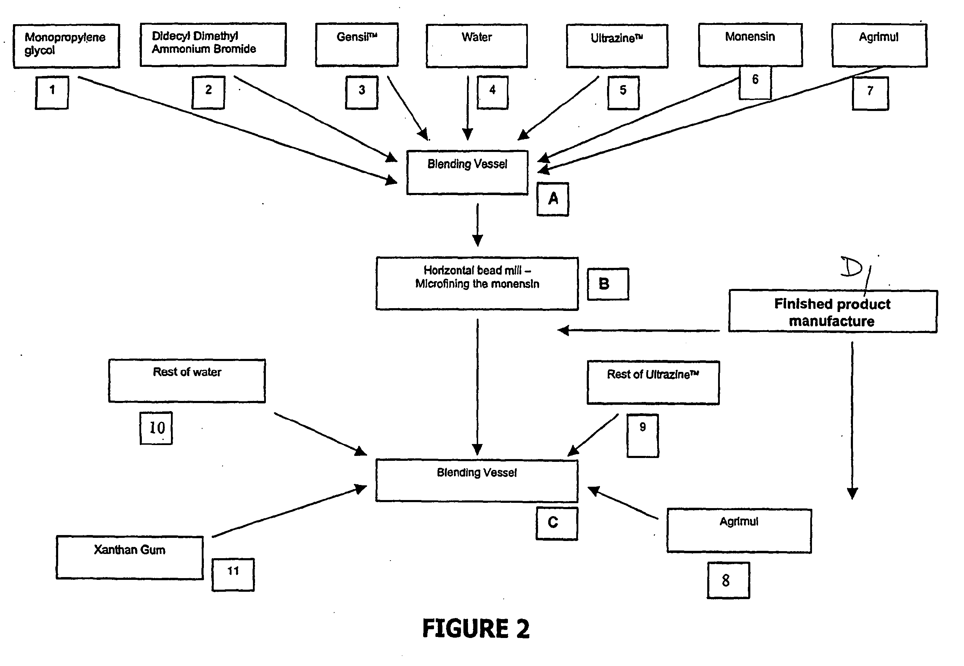

Preferred forms will now be described with reference to the following examples of which the mixing procedures utilised is preferably as depicted and sequenced in one of Figures 1 or 2.

The present invention recognises that having evolved a suitable stable aqueous suspension of microfine monensin a predictable in feed thereof to animals is possible simply by providing to the animal drinking water which includes the microfine ionophore antibiotic dispersed therein, said ionophore antibiotic having been included in the water supply by an intake directly or indirectly from a composition as previously defined. The less dilute the concentrate the more suspension agent we have found to be desirable.

Preferably the composition is for use in one or more of the trough treatment systems available.

**Reference Example 1**

| | |
|---|---|
| Sodium Monensin | 6.33% (about 6% monensin) w/v |
| Monopropylene glycol | 10% w/v |
| ULTRAZINE NA^{™} | 4-5% w/v |
| Disodium Phosphate Anhydrous | 0.355% w/v |
| MonoPotassium phosphate Dihydrate | 0.04% w/v |
| Dialkyl dimethyl ammonium bromide | .0064% w/v |
| Xanthan Gum | 0.4% w/v |
| Simethicone GENSIL^{™} | 0.333% w/v |
| Silicon Dioxide Antifoam | 0.167% w/v |
| Water to 100% | |

| | |
|---|---|
| Milling has resulted in a mean particle size for sodium monensin of 5 µm (microns). | |

**Example 2**

| | |
|---|---|
| Sodium Monensin | 6.33% (about 6% monensin) w/v |
| Monopropylene glycol | 10% w/v |
| Alkyl Polyglycoside | 0.5% w/v |
| Disodium Phosphate Anhydrous | 0.355% w/v |
| MonoPotassium phosphate Dihydrate | 0.04% w/v |
| Dialkyl dimethyl ammonium bromide | .0064% w/v |
| Xanthan Gum | 0.4% w/v |
| Simethicone GENSIL^{™} | 0.333% w/v |
| Silicon Dioxide Antifoam | 0.167% w/v |
| Water to 100% | |

| | |
|---|---|
| Milling has resulted in a mean particle size for sodium monensin of 5 µm (microns). | |

**Example 3**

| | |
|---|---|
| Sodium Monensin | 6.33% (about 6% monensin) w/v |
| Monopropylene glycol | 10% w/v |
| ULTRAZINE NA^{™} | 4-5% w/v |
| Disodium Phosphate Anhydrous | 0.355% w/v |
| MonoPotassium phosphate Dihydrate | 0.04% w/v |
| Dialkyl dimethyl ammonium bromide | .0064% w/v |
| Xanthan Gum | 0.4% w/v |
| Simethicone GENSIL^{™} | 0.333% w/v |
| Silicon Dioxide Antifoam | 0.167% w/v |
| Sorbitol as a Debittering agent | 3.5% w/v |
| Water to 100% | |

| | |
|---|---|
| Milling has resulted in a mean particle size for sodium monensin of 5 µm (microns). | |

**Example 4**

| | |
|---|---|
| Sodium Monensin | 6.33% (about 6% Monensin) w/v |
| Monopropylene glycol | 10% w/v |
| Alkyl Polyglycoside | 0.5% w/v |
| Disodium Phosphate Anhydrous | 0.355% w/v |
| MonoPotassium phosphate Dihydrate | 0.04% w/v |
| Dialkyl dimethyl ammonium bromide | .0064% w/v |
| Xanthan Gum | 0.4% w/v |
| Simethicone GENSIL^{™} | 0.333% w/v |
| Silicon Dioxide Antifoam | 0.167% w/v |
| Sorbitol as a Debittering agent | 3.5% w/v |
| Water to 100% | |

| | |
|---|---|
| Milling has resulted in a mean particle size for sodium monensin of 5 µm (microns). | |

**Example 5**

| **Ingredient name (common or chemical)** | **CAS number** | **Quantity (% w/w)** | **Function** |
|---|---|---|---|
| Sodium Monensin QA166H | 17090-79-8 | 6.747% | Active |
| Monopropylene glycol | 57-55-6 | 10% | Antifreeze |
| Didecyl dimethyl Ammonium Bromide | 2390-68-3 | 0.1 % | Preservative |
| Xanthan Gum | 11138-66-2 | 0.5% | Suspension agent |
| Sodium lignosulphonate | 8061-51-6 | 4.1% | Wetting agent |
| Alkyl Polyglycoside | 68515-73-1 | 3.8% | Wetting agent |
| Simethicone | 8050-81-5 | 0.333% | Antifoam |
| Silicon Dioxide | 7631-86-9 | 0.167% | Antifoam |
| Water balance q. v | 7732-18-5 | 74.47% | diluent |

Milling has resulted in a mean particle size for sodium monensin of 5 µm (microns).

Simethicone and Silicon dioxide together make up the proprietary brand "Gensil".

### 1. Preparation Procedure

The formulations of each of Examples 1 to 4 can be prepared by the procedure shown in Figure 1. The formulation of Example 5 is prepared by the procedure shown in Figure 2. The preferred method of preparing a formulation such as Reference Example 1 is as follows.

As can been seen from Figures 1 and 2 a blending vessel (A) which can, if desired, be the horizontal bead mill (B) but is preferably not, and a blending vessel (C) are utilised as the apparatus.

Most preferably however there is a three stage equipment base for the process viz. blending vessel (A), horizontal bead mill (B) for microfining the ionophore antibiotic and a blending vessel (C).

As can be seen from Figure 1 ingredients 1 through 6 are blended in the reference number sequence in the blending vessel (A) prior to passage into the horizontal bead mill (B). These pre-blended materials include: monopropylene glycol, dialkyl dimethyl ammonium bromide, GENSIL^{™} antifoam, some water, ULTRAZINE NA^{™} wetting agent, and monensin.

After the milling that premill mix, the product can be taken away either as an intermediate product (eg; post mill product) for subsequent use elsewhere for blending. Preferably however the output milled mix passes to blending vessel (C) where it is blended with the rest of the water (7), the remainder of the ULTRAZINE NA^{™} wetting agent (8) and the xanthan gum or other dispersion agent (9).

With a formulation whether to the formula of Reference Example 1 or Example 3 or another (Figures 1 and 2 do not refer to the buffering system nor to a debittering agent) very good suspensibility is obtained both of the concentrate and of a subsequent diluted form (eg; in a trough usage where the dilution is, for example, to about 3 to 6 ppm monensin).

The numerals 1-9 (Figure 1) or 1-11 (Figure 2) indicate the preferred sequence of ingredient addition. With reference to Figure 1, a pre-mill non sequenced mix of components 1 through 6 in the blending vessel (A) will still lead to a good mill mix yet is detrimental to the best suspensibility of the diluted form.

A formulation as in Reference Example 1 made by a procedure as in Figure 1 has a capability of being added as an aqueous concentrate into a large volume of water such as might be experienced in providing an infeed into a water system.

It is important to note the following processing issues:
the order of addition of the components to the grind base premix is not critical
the appropriate particle size induced by the grinding operation is critical
the order of addition of the components in the makeup tank is critical.

### 2. The Mill Mix

The mill mix is an important factor in the invention. It is the pre-mix of components which are added into the bead mill. The composition (identity and amount) is important in determining the ultimate particle size and the coatings on the particles which result.

The relative quantities (eg of MPG: monensin) are important in this respect.

### 3. Stability Data

### a) Shelf life stability

The following shelf file stability data indicates the 6% concentrate exhibits stability, at differing temperatures for at least 3 months (the length of time of the trials).

Trough Treatment 6% Concentrate Shelf Life Study

| | | | | |
|---|---|---|---|---|
| Batch number: I183 | | | | |
| 25°C Sample: | | | | |
| | Time 0 | 1 month | 2 months | 3 months |
| Appearance | *Normal | Normal | Normal | Normal |
| Coliforms | <1 | <1 | <1 | <1 |
| Monensin Biopotency | 5.7 | 5.5 | 5.4 | 5.6 |
| PH | 8.11 | 8.00 | 7.77 | 7.51 |
| Yeasts and Moulds | <1 | <1 | <1 | <1 |
| APC | <10 | <10 | <10 | <10 |
| | | | | |
| 42°C Sample : | | | | |
| | Time 0 | 1 month | 2 months | 3 months |
| Appearance | Normal | Normal | Normal | Normal |
| Coliforms | <1 | <1 | <1 | <1 |
| Monensin Biopotency | 5.7 | 5.6 | 5.4 | 5.4 |
| PH | 8.11 | 7.60 | 7.39 | 7.28 |
| Yeasts and Moulds | <1 | <1 | <1 | <1 |
| APC | <10 | <10 | <10 | <10 |

| | | | | |
|---|---|---|---|---|
| * Appearance-normal means light brown gelatinous liquid with brown specks present. | | | | |

### b) Positional Stability

The following experiments detail the positional stability of the trough treatment of the invention (TT).

Positional stability studies were conducted with a DOSATRON^{™} trough treatment system. This is a proportional feed system which delivers a % mix in proportion to demand. Such an administration system is able to spread delivery of a complete batch over 24 hours.

Figure 3 illustrates a dosatron system used in obtaining the positional stability data.

### Example 1

Trough treatment (TT) (600ppm) in a 200L plastic solution tank with a functioning Dosatron 8000.

Method: 100 litres water were added to the solution tank connected to a Dosatron 8000. 2 litres TT were mixed well with 2 litres water. This mix was added to the half-full solution tank, filled to the 200 litre level, mixed thoroughly. The Dosatron was set at 2 % and the water flow at 400 litres/hour to ensure the solution tank is completely used within 24 hours. The draw off tube from the Dosatron was set at a height 10 cm from the bottom of the tank.

Sampling: Samples for assay were taken from the draw-off taps. Before sampling, a 50 ml sample was drawn off and discarded. 1 x 100 ml samples are taken from each of the 4 taps set at either the top, middle or bottom of the tank. Samples will be taken at the specified time intervals. Each sample taken was individually identified and 50 ml from each sample taken and to a pooled sample (total Vol 200 ml). This sample was that assayed and the other samples retained. Samples were also taken from the four-tap set, here positioned in the plastic tank at a level 10 cm from the bottom of the tank. Samples were taken 0, 12, 24 hours after mixing.

After 24 hours a further 4 x 100 ml samples were taken from the bottom of the tank.

Each sample taken was individually identified and 50 ml from each sample taken and pooled (total Vol 200 ml). This sample was assayed and the other samples retained. In this case the drench gun tube attached to the rod was used.

### Example 2

Measurement of the positional stability of the TT (3000ppm) in a static 200 L plastic solution tank over a 4 day period.

Method: Before filling the tank, the bottom tap hoses were bent so the end was 5 cm from the bottom of the tank. 100 litres of water was added to the solution tank. 10 litres of TT was mixed well with 10 litres water. This mix was added to the half-full solution tank, filled to the 200 litre level and mixed.

Sampling: Samples for assay were taken from the draw-off taps. Before sampling, a 50 ml sample was drawn off and discarded. Each sample taken was individually identified and 50 ml from each sample taken and pooled (total Vol 200 ml). This sample was that assayed and the other samples retained. Samples were taken from the top, middle and bottom of the tank at the intervals of 0, 12 hrs, 24 hrs, 2 days and 4 days after mixing.

### Example 3

Measurement of the positional stability of TT (6 ppm) in a concrete trough over a 24 day period.

Method: 50 litres of water was added to the solution tank connected to the Dosatron 8000. 1 litre of TT was mixed well with 1 litre water. This mix was added to the half-full solution tank, filled to the 100 litre level and mixed. Set the Dosatron at 1% to ensure a trough concentration of 6 ppm. The trough was connected to the solution tank containing the Dosatron and TT. The new concrete trough was scrubbed clean and the water in the trough pH tested before use. A neutral pH (7-8) is acceptable. The water was discarded before filling from the Dosatron 8000.

Activation of the ball-cock filled the trough with TT (6 ppm). After filling the trough herd drinking was simulated by siphoning water from the top of the trough to activate the ball-cock. 5000 litres was siphoned from the trough over a 24 hour period.

Sampling: The draw-off tube from the drench gun was attached to a rigid pole.
The end of the draw off tube was set to sample the trough from 3 levels-top, middle and bottom. 4 x 100 ml samples was taken from each position. Each sample taken was individually identified and 50 ml from each sample taken and pooled (total Vol 200 ml). This sample was that assayed and the other samples retained. Sampling of the trough at the intervals of 0, 6 hrs, 12 hrs, 24 hrs, 5 days, 10 days and 24 days after mixing.

### Example 4

Measurement of the amount of Monensin settling in the drum after mixing TT at 3000 ppm and leaving for 4 days undisturbed.

Methods: 100 litres water was added to the solution tank connected to the Dosatron 8000. 10 litres of TT was mixed with 10 litres of water. This mix was added to the half-full solution tank, filled to the 200 litre level and mixed.

Sampling: Samples for assay were taken from the bottom of the tank. Before sampling, opened all middle taps and bottom taps to provide a slow release of tank mix. The rate of removal of the fluid was slow enough so the bottom of the tank was not disturbed. After the level of water fell below the bottom taps, mixed the bottom of the tank thoroughly and drew off 4 x 100 ml samples taken at different locations. Each sample taken was individually identified and 50 ml from each of the samples taken and pooled (total Vol 200 ml). The sample was that assayed and the other samples retained. N. B Please calculate the volume of water left in the drum before taking the samples. This will be to calculate the total amount of monensin settling in the bottom of the tank after 4 days.

### Positional stability results and discussion

The test had the following accuracy parameters:
Repeatability: The difference between results of duplicate portions of the same sample tested in the same run should not exceed 10% of the mean result. Recent results indicate that duplicate results are not exceeding 5.4% of the mean result.
Reproducibility: The difference between results of portions of the same sample tested at different times by different analysts should not exceed 15% of the mean result.

The repeatability and reproducibility data affords us quantifiable parameters for substantial homogeneity, indicating a substantially uniform suspension over time.

**Table 1: Example 1 Results (600 ppm)**

| **Time** | **Bottom Sample Results** |
|---|---|
| 0 hours | 556 ppm |
| 12 hours | 504 ppm |
| 24 hours | 439 ppm |

**Table 2: Example 1 Repeated Trial Results (600 ppm)**

| **Time** | **Bottom Sample Results** |
|---|---|
| 0 hours | 580 ppm |
| 12 hours | 470 ppm |
| 24 hours | 420 ppm |

**Table 3: Example 2 Results (3000 ppm)**

| **Time** | **Top Sample (ppm)** | **Middle Sample (ppm)** | **Bottom Sample (ppm)** |
|---|---|---|---|
| 0 hours | 2970 | 2900 | 3030 |
| 12 hours | 2680 | 2720 | 2760 |
| 24 hours | 2930 | 2970 | 3060 |
| 2 days | 2960 | 2950 | 3030 |
| 4 days | 2190 | 2930 | 3050 |

**Table 4: Example 3 Results (6 ppm)**

| **Time** | **Top Sample (ppm)** | **Middle Sample (ppm)** | **Bottom Sample (ppm)** |
|---|---|---|---|
| 0 hours | 5.0 | 5.5 | 5.1 |
| 12 hours | 4.9 | 4.6 | 5.2 |
| 24 hours | 4.7 | 4.9 | 5.0 |
| 2 days | 5.6 | 5.2 | 5.0 |
| 4 days | 5.1 | 5.3 | 4.3 |
| 10 days | 5.5 | 5.1 | 5.8 |
| 24 days | 5.0 | 5.0 | 5.3 |

## Claims

1. An aqueous suspension of at least one ionophore antibiotic capable of subsequent dilution, said suspension comprising:
• a milled product comprising an ionophore antibiotic or ionophore antibiotics milled with at least the following dispersing agents:
(i) a glycol and
(ii) at least one of a lignosulfonate and a polyglycoside,
wherein the ionophore antibiotic or ionophore antibiotics have a mean particle size of less than 20 µm (microns), and
• at least water and a suspension agent selected from xanthan gum and guar gum.

2. The suspension according to claim 1 wherein the ionophore antibiotic or ionophore antibiotics have a mean particle size of less than 5 µm (microns).

3. The suspension according to claim 1 or claim 2 wherein the ionophore antibiotic or ionophore antibiotics are in the crystalline form.

4. The suspension according to any one of claims 1 to 3 wherein the ionophore antibiotic is monensin.

5. The suspension according to any one of claims 1 to 4 wherein the glycol is a liquid.

6. The suspension according to any one of claims 1 to 5 wherein the glycol is monopropylene glycol.

7. The suspension according to any one of claims 1 to 6 wherein the milled product comprises a polyglycoside.

8. The suspension according to claim 7 wherein the polyglycoside is alkyl polyglycoside.

9. The suspension according to any one of claims 1 to 8 wherein the milled product comprises a lignosulfonate.

10. The suspension according to any one of claims 1 to 9 wherein the milled product further comprises an antifoam agent.

11. A method of forming an aqueous suspension of at least one ionophore antibiotic capable of subsequent dilution, said method comprising:
• milling an ionophore antibiotic or ionophore antibiotics with at least the following dispersing agents:
(i) a glycol and
(ii) at least one of a lignosulfonate and a polyglycoside,
wherein the ionophore antibiotic or ionophore antibiotics are reduced to a mean particle size of less than 20 µm (microns) and the milling is in liquid supported conditions, and
• subsequently formulating the suspension with at least water and a suspension agent selected from xanthan gum and guar gum.

12. The method according to claim 11 wherein the ionophore antibiotic or ionophore antibiotics are reduced to a mean particle size of less than 5 µm (microns).

13. The method according to claim 11 or 12 wherein the ionophore antibiotic or ionophore antibiotics are in the crystalline form.

14. The method according to any one of claims 11 to 13 wherein the ionophore antibiotic is monensin.

15. The method according to any one of claims 11 to 14 wherein the glycol is a liquid.

16. The method according to any one of claims 11 to 15 wherein the glycol is monopropylene glycol.

17. The method according to any one of claims 11 to 16 wherein the milling is in the presence of a polyglycoside.

18. The method according to claim 17 wherein the polyglycoside is alkyl polyglycoside.

19. The method according to any one of claims 11 to 18 wherein the milling is in the presence of a lignosulfonate.

20. The method according to any one of claims 11 to 19 wherein the milling is in the presence of an antifoam agent.

## Patentansprüche

1. Wässrige Suspension mindestens eines lonophorantibiotikums mit der Fähigkeit zur nachfolgenden Verdünnung, wobei die Suspension die folgenden Bestandteile umfasst:
- ein gemahlenes Produkt, das ein lonophorantibiotikum oder lonophorantibiotika umfasst, die mit mindestens den folgenden Dispergiermitteln vermahlen sind:
(i) einem Glycol und
(ii) mindestens einem Bestandteil aus einem Lignosulfonat und einem Polyglycosid,
wobei das lonophorantibiotikum oder die lonophorantibiotika eine mittlere Teilchengröße von weniger als 20 µm (microns) aufweisen und
- mindestens Wasser und ein Suspendiermittel, das aus Xanthanlösung und Guarmehl ausgewählt ist.

2. Suspension nach Anspruch 1, wobei das lonophorantibiotikum oder die lonophorantibiotika eine mittlere Teilchengröße von weniger als 5 µm (microns) aufweisen.

3. Suspension nach Anspruch 1 oder Anspruch 2, wobei das lonophorantibiotikum oder die lonophorantibiotika in kristalliner Form vorliegen.

4. Suspension nach einem der Ansprüche 1 bis 3, wobei das lonophorantibiotikum Monensin ist.

5. Suspension nach einem der Ansprüche 1 bis 4, wobei das Glycol eine Flüssigkeit ist.

6. Suspension nach einem der Ansprüche 1 bis 5, wobei das Glycol Monopropylenglycol ist.

7. Suspension nach einem der Ansprüche 1 bis 6, wobei das gemahlene Produkt ein Polyglycosid umfasst.

8. Suspension nach Anspruch 7, wobei das Polyglycosid ein Alkylpolyglycosid ist.

9. Suspension nach einem der Ansprüche 1 bis 8, wobei das gemahlene Produkt ein Lignosulfonat umfasst.

10. Suspension nach einem der Ansprüche 1 bis 9, wobei das gemahlene Produkt ferner ein Antischaummittel umfasst.

11. Verfahren zur Ausbildung einer wässrigen Suspension mindestens eines lonophorantibiotikums mit der Fähigkeit zur nachfolgenden Verdünnung, wobei das Verfahren die folgenden Stufen umfasst:
- Mahlen eines lonophorantibiotikums oder von lonophorantibiotika mit mindestens den folgenden Dispergiermitteln:
(i) einem Glycol und
(ii) mindestens einem Bestandteil aus einem Lignosulfonat und einem Polyglycosid,
wobei das lonophorantibiotikum oder die lonophorantibiotika auf eine mittlere Teilchengröße von weniger als 20 µm (microns) reduziert werden und das Mahlen in flüssigen geträgerten Zuständen erfolgt, und
- nachfolgendes Formulieren der Suspension mit mindestens Wasser und einem Suspendiermittel, das aus Xanthanlösung und Guarmehl ausgewählt ist.

12. Verfahren nach Anspruch 11, wobei das lonophorantibiotikum oder die lonophorantibiotika auf eine mittlere Teilchengröße von weniger als 5 µm (microns) reduziert werden.

13. Verfahren nach Anspruch 11 oder 12, wobei das lonophorantibiotikum oder die lonophorantibiotika in kristalliner Form vorliegen.

14. Verfahren nach einem der Ansprüche 11 bis 13, wobei das lonophorantibiotikum Monensin ist.

15. Verfahren nach einem der Ansprüche 11 bis 14, wobei das Glycol eine Flüssigkeit ist.

16. Verfahren nach einem der Ansprüche 11 bis 15, wobei das Glycol Monopropylenglycol ist.

17. Verfahren nach einem der Ansprüche 11 bis 16, wobei das Mahlen in Gegenwart eines Polyglycosids erfolgt.

18. Verfahren nach Anspruch 17, wobei das Polyglycosid ein Alkylpolyglycosid ist.

19. Verfahren nach einem der Ansprüche 11 bis 18, wobei das Mahlen in Gegenwart eines Lignosulfonats erfolgt.

20. Verfahren nach einem der Ansprüche 11 bis 19, wobei das Mahlen in Gegenwart eines Antischaummittels durchgeführt wird.

## Revendications

1. Suspension aqueuse d'au moins un antibiotique ionophore qui peut être soumise à une dilution ultérieure, ladite suspension comprenant :
• un produit broyé comprenant un antibiotique ionophore ou des antibiotiques ionophores broyés avec au moins les agents de mise en dispersion suivants :
(i) un glycol ; et
(ii) au moins soit un lignosulfonate, soit un polyglycoside ; l'antibiotique ionophore ou les antibiotiques ionophores possédant une granulométrie moyenne inférieure à 20 µm (microns) ; et
• au moins de l'eau et un agent de mise en suspension choisi parmi la gomme de xanthane et la gomme de guar.

2. Suspension selon la revendication 1, dans laquelle l'antibiotique ionophore ou les antibiotiques ionophores possèdent une granulométrie moyenne inférieure à 5 µm (microns).

3. Suspension selon la revendication 1 ou 2, dans laquelle l'antibiotique ionophore ou les antibiotiques ionophores sont sous forme cristalline.

4. Suspension selon l'une quelconque des revendications 1 à 3, dans laquelle l'antibiotique ionophore est la monensine.

5. Suspension selon l'une quelconque des revendications 1 à 4, dans laquelle le glycol est un liquide.

6. Suspension selon l'une quelconque des revendications 1 à 5, dans laquelle le glycol est du monopropylèneglycol.

7. Suspension selon l'une quelconque des revendications 1 à 6, dans laquelle le produit broyé comprend un polyglycoside.

8. Suspension selon la revendication 7, dans laquelle le polyglycoside est un alkylpolyglycoside.

9. Suspension selon l'une quelconque des revendications 1 à 8, dans laquelle le produit broyé comprend un lignosulfonate.

10. Suspension selon l'une quelconque des revendications 1 à 9, dans laquelle le produit broyé comprend en outre un agent antimousse.

11. Procédé de préparation d'une suspension aqueuse d'au moins un antibiotique ionophore qui peut être soumise à une dilution ultérieure, ledit procédé comprenant :
• le broyage d'un antibiotique ionophore ou d'antibiotiques ionophores avec au moins les agents de mise en dispersion suivants :
(i) un glycol ; et
(ii) au moins soit un lignosulfonate, soit un polyglycoside ; l'antibiotique ionophore ou les antibiotiques ionophores étant réduits à une granulométrie moyenne inférieure à 20 µm (microns) et le broyage se déroulant dans des conditions supportées par un liquide ; et
• la formulation ultérieure de la suspension avec au moins de l'eau et un agent de mise en suspension choisi parmi la gomme de xanthane et la gomme de guar.

12. Procédé selon la revendication 11, dans lequel l'antibiotique ionophore ou les antibiotiques ionophores sont réduits à une granulométrie moyenne inférieure à 5 µm (microns).

13. Procédé selon la revendication 11 ou 12, dans lequel l'antibiotique ionophore ou les antibiotiques ionophores sont sous forme cristalline.

14. Procédé selon l'une quelconque des revendications 11 à 13, dans lequel l'antibiotique ionophore est la monensine.

15. Procédé selon l'une quelconque des revendications 11 à 14, dans lequel le glycol est un liquide.

16. Procédé selon l'une quelconque des revendications 11 à 15, dans lequel le glycol est du monopropylèneglycol.

17. Procédé selon l'une quelconque des revendications 11 à 16, dans lequel le broyage se fait en présence d'un polyglycoside.

18. Procédé selon la revendication 17, dans lequel le polyglycoside est un alkylpolyglycoside.

19. Procédé selon l'une quelconque des revendications 11 à 18, dans lequel le broyage se fait en présence d'un lignosulfonate.

20. Procédé selon l'une quelconque des revendications 11 à 19, dans lequel le broyage se fait en présence d'un agent antimousse.
